# EUROPEAN PATENT APPLICATION

(11) **EP 3 878 446 A1**
(43) Date of publication of application: **15.09.2021**
(21) Application number: 20161715.6
(22) Date of filing: 09.03.2020
(51) Int. Cl.: A61K 31/225, A61K 31/4196, A61K 31/44, A61K 31/451, A61K 39/395, A61K 45/06, A61P 35/00, A61P 35/02

(54) **HSD11B1 INHIBITORS FOR USE IN IMMUNOTHERAPY AND USES THEREOF**

(71) Applicant: UNIVERSITE DE GENEVE, 1211 Geneva 4 (CH)
(72) Inventor: POMMIER, Aurélien, 74800 LA ROCHE SUR FORON (FR); BOURQUIN, Carole, 1782 BELFAUX (CH)
(74) Representative: reuteler & cie SA

(57) **Abstract**

The present invention relates to HSD11B1 inhibitors useful for increasing anti-tumor immune response in combination with immunotherapy, in particular for the treatment of cancers and to related compositions and methods using those.

SEQUENCE LISTING

<110>  UNIVERSITE DE GENEVE

<120>  HSD11B1 INHIBITORS FOR USE IN IMMUNOTHERAPY AND USES THEREOF

<130>  P2447EP00

<160>  3

<170>  PatentIn version 3.5

<210>  1
<211>  8
<212>  PRT
<213>  Gallus gallus domesticus

<400>  1

Ser Ile Ile Asn Phe Glu Lys Leu
1               5


<210>  2
<211>  23
<212>  DNA
<213>  Artificial sequence

<220>
<223>  gRNA targeting mouse hsd11b

<400>  2
gctgggcaca agttcgagtt agg                                               23


<210>  3
<211>  23
<212>  DNA
<213>  Artificial sequence

<220>
<223>  gRNA targeting mouse hsd11b1

<400>  3
atggtggtca tcttggtcgt agg                                               23

## Description

### Field of the invention

The present invention relates to the agents useful in combination with immunotherapy, in particular for increasing responsiveness to immunotherapy treatments. The invention further relates to methods and compositions useful in the treatment of cancers, in particular solid tumor cancers.

### Background

Immunotherapy of cancer aiming to stimulate the immune system against tumor cells has seen unprecedented success in recent years. The term immunotherapy regroups several therapeutic approaches including the use of immune checkpoint inhibitors, cell transfer therapies, monoclonal antibodies, treatment vaccines, immune system modulators and immunoconjugates. Although the use of immunotherapy is increasing, an objective clinical response is still observed only in a minority of patients. The determinant factors driving resistance to immunotherapy are not fully understood and the identification of immunosuppressive molecular pathways able to reduce the efficacy of immunotherapy is of high interest to improve clinical outcome of patients with cancer. Biological processes critical to antitumour immunity, such as interferon signalling, antigen presentation (Kalbasi et al., 2020, Nat Rev Immunol, 20(1), 25-39*),* loss of MHCI or mutation in beta-2 microglobulin and PD-L1 (Programmed death-ligand 1) expression in tumors (Syn, et al., 2017, Lancet Oncol, 18(12), e731-e741) have been described as important factors involved in sensitivity to immunotherapy. Other efforts have shed light on the immunological implications of canonical cancer signalling pathways, such as WNT-β- catenin signalling, cell cycle regulatory signalling, mitogen- activated protein kinase signalling and pathways activated by loss of the tumour suppressor phosphoinositide phosphatase PTEN (Phosphatase and tensin homologue on chromosome 10) in the context of immune checkpoint inhibitors treatment (*Kalbasi et al., 2020, supra*)*.* In the field of adoptive cell therapies, growing experience with these agents has revealed that limitations to durable remissions after chimeric antigen receptor (CAR) T cell therapy includes poor CAR T cell persistence in leukemia (Shah et al., 2019, Nat Rev Clin Oncol, 16(6), 372-385)*.* CAR T cell persistence is also considered as an important obstacle to overcome in order to reach efficacy of adoptive T cell therapies in solid tumors (*Shah et al., 2019, supra*)*.*

Acquired resistance to checkpoint inhibition such as anti-PD-1 therapy is also a problem, with approximately one quarter of responders who later present a disease progression (Ribas et al., 2016, JAMA, 315:1600-9).
Therefore, it is critical to identify and target novel biological mechanisms involved in resistance to immunotherapy.

Endogenous glucocorticoids are steroid hormones derived from cholesterol, which have diverse physiological effects. In human, cortisol is the main active glucocorticoid which controls several biological mechanisms in cells by binding to the glucocorticoid receptor. The activated glucocorticoid receptor-cortisol complex up-regulates the expression of target genes in the nucleus (a process known as transactivation) and represses the expression of genes involved in inflammation and immune response by preventing the translocation of other transcription factors such as NF-kB and API (transrepression). Glucocorticoid receptor is expressed in key immune cells involved in anti-tumor immunity such as T cells (Purton et al., 2004, J. Immunol, 173(6), 3816-24)*,* dendritic cells and macrophages (Heasman et al., 2003, J. Endocrinol, 178(1), 29-36)*.*
The activation of glucocorticoid receptor depends on the regulation of cortisol bioavailability. At systemic level, glucocorticoids are first secreted by the adrenal gland in response to hypothalamic pituitary stimulation. Second, the human liver/splanchnic bed contributes between 20 and 40% of daily cortisol production (Andrew et al., 2002, J. Clin. Endocrinol. Metab., 87(1), 277-85*;* Basu et al., 2004, Diabetes, 53(8), 2051-9*)* thus making a major impact on the half-life of cortisol. In tissues, the level of active cortisol can be controlled by 11-beta-hydroxysteroid dehydrogenase Type 1 (HSD11B1) and Type 2 (HSD11B2) enzymes. HSD11B1 is a low affinity enzyme with Km for cortisone in the micromolar range that prefers NADPH/NADP- (nicotinamide adenine dinucleotide phosphate) as cofactors. HSD11B1 is widely expressed with a distribution similar reported in rodents, non-human primates and humans (Agarwal et al., 1989, J. Biol. Chem, 264(32),18939-43*;* Moisan et al., 1990, J. Neuroendocrinol, 2(6), 853-8*;* Tannin et al., 1991, J. Biol. Chem, 266(25), 16653-8*).*

HSD11B1 expression is found in liver (*Tannin et al., 1991, supra;* Moisan et al., 1990, Endocrinology, 127(3), 1450-5), brain (*Moisan et al., 1990, supra;* Lakshmi, et al., 1991, Endocrinology, 128(4), 1741-8*),* uterus (Burton et al., 1998, Endocrinology, 139(1), 376-82*),* placenta (Waddell et al., 1998, Endocrinology, 139(4), 517-23), adipose tissues (Bujalska et al., 1997, Lancet, 349(9060): p. 1210-3*),* skeletal muscle (Whorwood et al., 2002, Diabetes, 51(4), 1066-75*),* heart (Walker et al., 1991, Endocrinology, 129(6), 3305-12) and immune and inflammatory cells (Gilmour et al., 2006, J. Immunol., 176(12), 7605-11)*.* Most studies have shown that HSD11B1 functions primarily as a reductase in intact cells converting inactive 11-ketoglucocorticoids (i.e., cortisone or dehydrocorticosterone) to active 11-hydroxycorticoids (i.e., cortisone or corticosterone), and thereby amplifies glucocorticoid action in a tissue-specific manner.

Importantly, glucocorticoids have both anti-inflammatory and immunosuppressive activity. The intensity of their immunomodulatory effect depends not only on circulating levels but also on the tight regulation at the pre-glucocorticoid receptor level where HSD11B1 and HSD11B2 have a critical impact.

In recent years, glucocorticoids have gained interest in the field of cancer research. Studies in breast, prostate and ovarian cancer have shown a correlation between high levels and activity of GR in tumors and poor outcome (Arora et al., 2013, Cell, 155(6),1309-22*;* Pan et al., 2011, Cancer Res, 71(20), 6360-70*;* Veneris et al., 2017, 146(1), 153-160).

HSD11B2 catalyses the conversion of cortisol to the inactive metabolite cortisone modulating thereby the intracellular glucocorticoid levels. HSD11B2 is mainly expressed and active in the kidney and its primary function is to protect the nonselective mineralocorticoid receptor from occupation by glucocorticoids. Cirillo et al., 2017, British Journal of Cancer, 117, 984-993 raised the possibility that different tumor cell types can produce cortisol and that it was possible to modulate the bioavailability of cortisol by manipulation of HSD11B2.

Zhang et al., 2005, J. Immunol, 174(2), 879-89 demonstrate that inhibition of HSD11B1 in T cell increases their activation levels, while Rocamora-Reverte et al. 2017, Cell Death Dis, 8(7), e2948 showed that activation of glucocorticoids by HSD11B1 in T cells participates to autonomous cell death.

Inhibition of 11-beta-hydroxysteroid dehydrogenase enzymes in a living system has been described for the treatment of various conditions by the administration of an inhibitor of conversion of cortisol-to-cortisone or of an inhibitor of conversion of cortisone-to-cortisol (WO 2004/027047). WO 2006/097337 described the generation of specific inhibitors against HSD11B1 and/or HSD11B2 to either selectively or combined inhibition of the enzymes and to allow fine tuning of local cortisol levels to compensate for cortisol excess or deficiencies. It indicated that the use of inhibitors against HSD11B2 would be preferred for the treatment of cancer and/or cell proliferation.

The preferred inhibition of HSD11B2 claimed in WO 2006/097337 was based on the direct antiproliferative effect of cortisol on tumor cells. Indeed, since HSD11B2 could provide an enzymatic shield that may protect the tumor cells from the antiproliferative effects of the glucocorticoids, the specific inhibition of HSD11B2 over HSD11B1 was preferred.

Based on the low number of patients currently responding to immunotherapy (only about 20-40% of patients respond to immunotherapy as reported by Sharma et al., 2017, Cell 168(4), 707-723*),* there is still a critical need to discover novel therapeutic methods to improve the efficacy of immunotherapy that would overcome these shortcomings.

### Summary of the Invention

The invention is based on the unexpected finding that resistance to immunotherapy can derive from a reduced efficacy of the anti-tumor immune response which is induced by the enzymatic conversion of inactive 11-ketoglucocorticoids (i.e., cortisone or dehydrocorticosterone) to active 11-hydroxycorticoids (i.e., cortisone or corticosterone). The invention further relates to the finding that the use of selective inhibitors of HSD11B1 in combination with immunotherapy induces an increased response to immunotherapy, which would be beneficial for the treatment of cancer.
Therefore, the invention further relates to a newly developed combination, which has a surprisingly effective anticancer activity, and would be useful in preventing intrinsic or acquired resistance to immunotherapy in particular in malignant and resistant cancers such as melanoma, lung carcinoma, glioblastoma, renal carcinoma, gastrointestinal stromal tumor and leukemia.

According to one aspect of the invention, is provided a HSD11B1 inhibitor for use in the treatment of a cancer, wherein said HSD11B1 inhibitor is to be administered in combination with immunotherapy.
According to another aspect of the invention, is provided a combination of at least one HSD11B1 inhibitor and at least one agent useful in immunotherapy for use in the treatment of a cancer.
According to another aspect of the invention, is provided a use of a combination of the invention for the preparation of a pharmaceutical composition for the treatment of cancer.

According to another aspect of the invention, is provided a pharmaceutical composition comprising at least one HSD11B1 inhibitor, at least one agent useful in immunotherapy and at least one pharmaceutically acceptable carrier and its use as a medication.
According to another aspect of the invention, is provided a method for treating a subject who is suffering from a cancer, said method comprising administering a therapeutically effective amount of an agent inducing HSD11B1 inhibition or any suitable pharmaceutically acceptable formulation thereof, in a subject in need thereof in combination with immunotherapy.
In another embodiment, the invention provides a method of identifying agents useful in the potentiation of immunotherapy, said method comprising identifying agents that are able to inhibit HSD11B1, in particular as described herein.

### Description of the figures

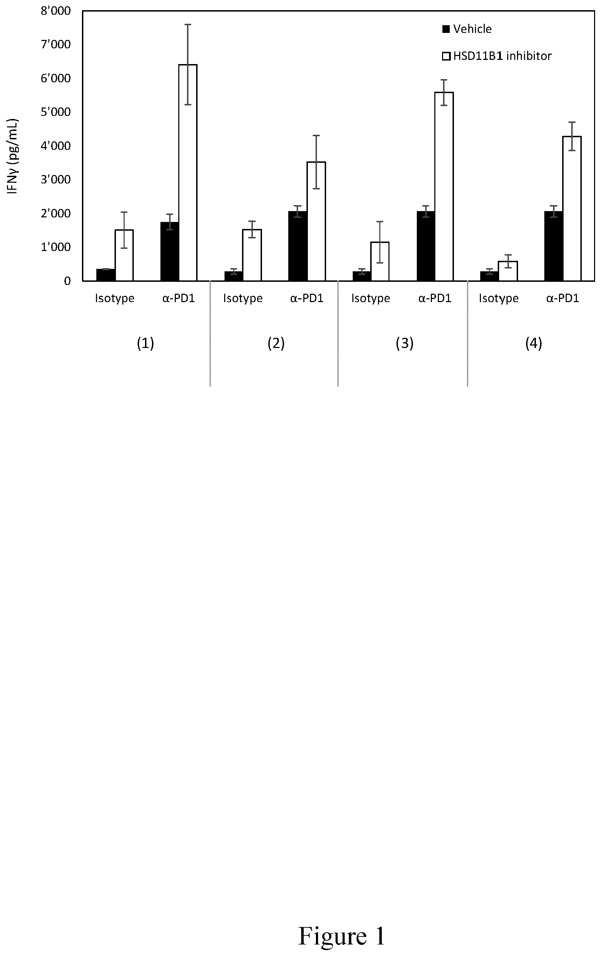

**Figure 1** represents the levels of IFNγ measured in PBMC supernatant after the combined use of various HSD11B1 inhibitors (BMS-823778 **(1),** PF-915275 **(2),** 10J **(3)** and carbenoxolone **(4)**) with anti-PD-1 immunotherapy (G4K) in human immune cells compared to vehicle and isotype control, as described in Example 1.

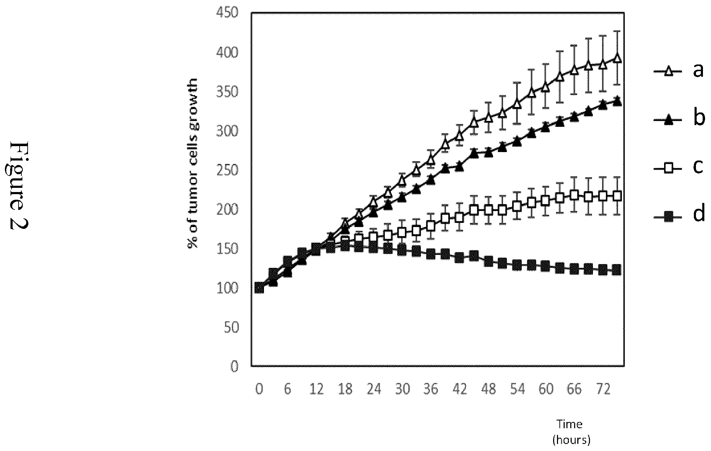

**Figure 2** represents growth of tumor cells cultured with immune cells and the combination of: **a:** vehicle control and isotype; **b:** HSD11B1 inhibitor **(1)** and isotype; **c:** vehicle control and anti-PD-1 immunotherapy (G4K) or **d:** HSD11B1 inhibitor **(1)** and anti-PD-1 immunotherapy (G4K) as described in Example 2.

### Detailed description

The expression "pharmaceutically acceptable salts" refers to salts of the below-specified compounds. Examples of such salts include, but are not restricted, to base addition salts formed by reaction of those compounds with organic or inorganic bases or inorganic acids (e.g. hydrochloric acid and the like).
As used herein, "treatment" and "treating" and the like generally mean obtaining a desired pharmacological and physiological effect. The effect may be prophylactic in terms of preventing or partially preventing a disease, symptom or condition thereof and/or may be therapeutic in terms of a partial or complete cure of a disease, condition, symptom or adverse effect attributed to the disease. The term "treatment" as used herein covers any treatment of a melanoma in a mammal, particularly a human, and includes inhibiting the disease, i.e., arresting its development; or relieving the disease, i.e., causing regression of the disease and/or its symptoms or conditions such as improvement or remediation of damage.

The term "efficacy" of a treatment or method according to the invention can be measured based on changes in the course of disease or condition in response to a use or a method according to the invention. According to a particular embodiment, the efficacy of a combined use according to the invention can be measured through the assessment of efficacy of a compound combination of the invention, in particular long term efficacy regarding repression of tumor growth, progression and dissemination, decrease of the number of cancer cells or their proliferation rate in combination with immunotherapy as compared to the efficacy of each compound of the combination, taken alone. According to another embodiment, the efficacy of a combined use according to the invention can be assessed by the monitoring of immune activation (eg. IFN y expression in peripheral blood) the observation of progression free survival (defined by the RECIST criteria) and overall survival in human patients.
According to another aspect, the efficacy of a combined use according to the invention can be assessed for example through the observation of IFNγ secretion level for example by ELISA in an antigen recall assay using peripheral blood mononuclear cells (PBMC) isolated from healthy donors or by assessing the immune cell killing potential of T cells against cancer cells in mouse as described herein.
The term "subject" as used herein refers to mammals. For examples, mammals contemplated by the present invention include human, primates, domesticated animals such as cattle, sheep, pigs, horses, laboratory rodents, other pets and the like.
The expression "11-beta-hydroxysteroid dehydrogenase Type 1 (HSD11B1) inhibitor" refers to agents able to inhibit HSD11B1. Inhibition of HSD11B1 can be assessed for example by measuring the enzymatic transformation of HSD11B1 substrates (11-DHC or cortisone) into products (corticosterone or cortisol) by ELISA or mass spectrometry or in yeast cell based assays described Vanella et al., 2016, Microb Cell Fact., 15: 52, or cell-based assays on human 11β-HSD1 transfected HEK-293 cell line with an Scintillation Proximity Assay (WO 2006/100502) or by molecular modelling of the crystal structure of one subunit of human 11β-HSD1 such as described by Quian et al., Molecules, (9). pii: E1222*.* Inhibition of HSD11B1 can be also assessed at the functional level for example by testing the inhibitory effect of immune suppression of HSD11B1 products on immune cells as described in this invention. HSD11B1 inhibitors can be small molecules inhibitors, peptides, mAbs, chimeric proteins or fusion proteins, aptamers (including peptide aptamers, DNA and RNA aptamers), soluble receptors and such agents may be acting by silencing, or down-regulating HSD11B1 at the genetic level by nucleic acid interaction, nucleic acid mutations or deletion aiming to abrogate the enzymatic activity of HSD11B1 using CRISPR-Cas9 technologies or by systemic administration of RNA interference delivered by liposomes or nanoparticles in human. HSD11B1 inhibitors can be administered in humans or on isolated immune cells in case of adoptive transfer therapy (e.g. CAR T cells).
Among those agents, some HSD11B1 inhibitors were actively developed for the treatment of different diseases including diabetes, metabolic diseases, obesity, Alzheimer, intraocular hypertension, arteriosclerosis or NAFLD (Non-Alcoholic Fatty Liver Disease) by several companies. All of these compounds failed in clinical trials so far. Some of them are still on clinical trial (AZD4017, from AstraZeneca in Phase II for intra ocular pressure intracranial hypertension). None of them has been tested in oncology. For example, HSD11B1 inhibitors according to the invention have been described in Table 1 below.

**Table 1**

| **Chemotypes** | **Common names of examples of specific compounds** | **Description of the chemotypes** | |
|---|---|---|---|
| 3,4,5 -trisubstituted -4-1,2,4-triazole | MK-0736 | Compounds described in PCT/WO2010/68580, in particular compounds of the following structure as described in PCT/US2004/133011 and PCT/US2009/066939, in particular, compounds of the following structures | |
| | | 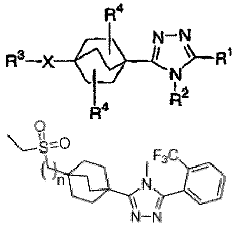 | |
| | | 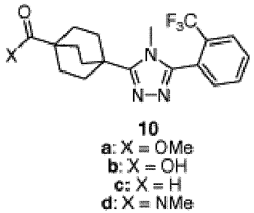 | |
| | | As described in Maletic et al., 2011, Bioorg Med Chem Lett. 2011, 21(8):2568-72 | |
| | BMS 823778 | Compounds of the following structure as described in PCT/US/2011/288051 | |
| | | 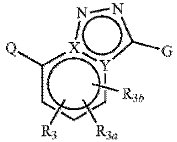 | |
| | | and in particular compounds of the following structure | |
| | | 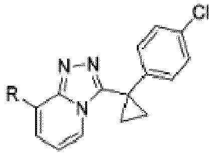 | as described in *Li* |
| | | *et al.,* 2018, ACS Med Chem Lett., 9(12):1170-1174 | |
| | MK0916 | Compounds of the following structures as described in PCT/US/2004/004891, PCT/US/2004/0106664 PCT/US/2005/001928 | |
| | | 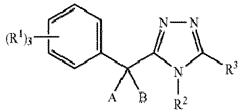 | |
| | | 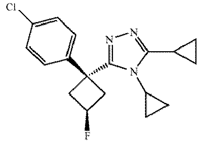 | |
| N-(thiazol-2-yl) benzenesulfonamide | BVT2733 | Compounds described in PCT/SE2001/01155 PCT/WO 2001/090090 and in particular compounds of the following structure | |
| | | 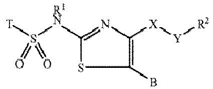 | as described in |
| | | PCT/US/2010/0113435 | |
| | BVT-3498 | Compounds described in PCT/SE/2001/01155, and PCT/US/2010/011343 and in particular compounds of the following structure | |
| | | 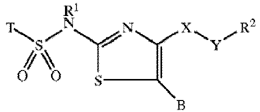 | |
| | | as described in PCT/US/2004/0224996 | |
| S-(1-H-pyrazol-4-yl)thiophene-3-carboxamide | UE2343 | Compounds of the following structure as described in Webster, 2017, british journal of pharmacology, 174(5):396-408*.* | |
| | | 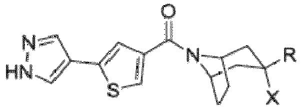 | |
| Spirocyclic ureas | 10J | Compounds of the following structure as described in PCT/US2007/018789 | |
| | | 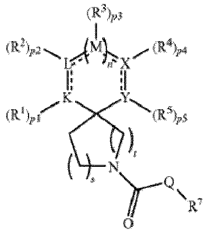 | |
| | | In particular of the following structure | |
| | | 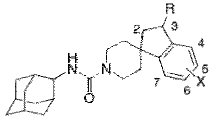 | |
| | | as described in Tice et al., 2010, Bioorg. Med. Chem. Lett., 20(3):881-6 | |
| Adamantanes | ABT-384 | Compounds of the following structures as described in PCT/US2005/245534, PCT/US/2005/277647 PCT/WO/2009/026422, PCT/US/2010/222316 and Rhode et al., 2006, Bioorganic & Medicinal Chemistry Letters, 16(23):5958-5962 | |
| | | 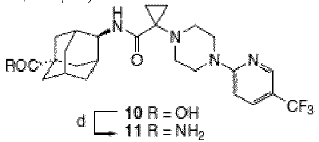 | |
| | | 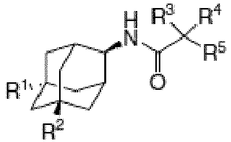 | |
| Carbamoyl | AZD8329 | Compounds described in PCT/US/2009/022 1660 PCT/US/2011/002853 or PCT/US/2011/0028529, PCT/US/2011/022427, in particular compounds of the following structure as PCTUS/2008/0269288, PCT/US/2009/312372, PCT/US/2009/030607 | |
| | | 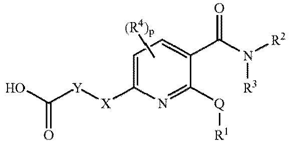 | |
| | | or of the following structures as described in PCT/US/20110224273 or Scott et al., 2012, J. Med. Chem., 55, 22, 10136-10147 | |
| | | 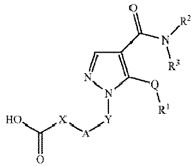 | |
| | | 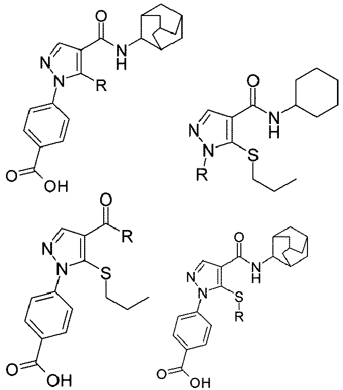 | |
| Thiazolones | AMG-221 | Compounds as described in PCT/WO/2009/002445, PCT/WO/2010/008729, PCT/WO/2010/014586, PCT/WO/2012/051139, in particular, compounds of the following structures | |
| | | 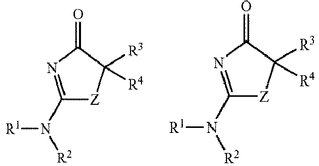 | |
| | | 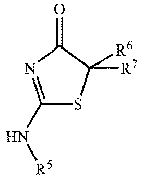 | |
| | | as described in PCT/US/2008/045503, in particular compounds of the following structures | |
| | | 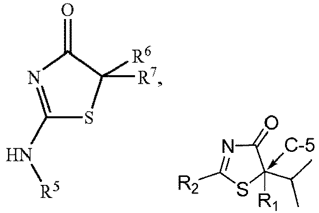 | |
| | | as described in Veniant et al., 2010, J. Med. Chem. 2010, 53, 11, 4481-4487 | |
| Arylsulfonamide | PF-915275 | Compounds of the following structure | |
| | | 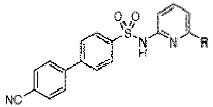 | |
| | | as described in PCT/US/2007/0072914 or in Siu et al., 2009, Bioorg Med Chem Lett., 19(13):3493-7 | |
| Cyclohexylcarbamoyl | AZD4017 | Compounds of the following structures as described in PCT/US/2008/0269288, PCT/US/2009/312372 and PCT/US/2009/030607 | |
| | | 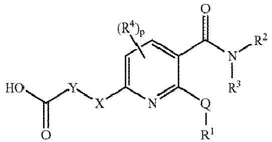 | |
| | | 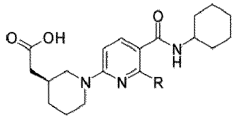 | |
| | | or *in* Scott, et al., 2012, Journal of medicinal chemistry or having the following structures | |
| | | 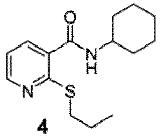 | |
| | | 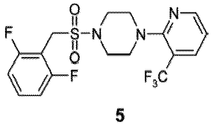 | |
| Sulfonamides | HSD-016 | Compounds of the following structure as described in PCT/US/2010/029648, PCT/US/2007/0219198 | |
| | | 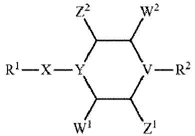 | |
| Glycyrrhetinic acid | | Compounds of the following structure | |
| derivatives | | 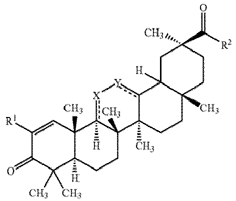 as described in | |
| | | PCT/CA/2007/001127 | |
| | BI 135585 | Compounds of the following structures as described in PCT/US2011/040443, PCT/US/2013/0244993 | |
| | | 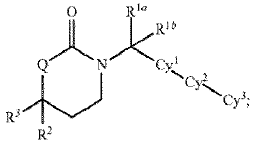 | |
| | | 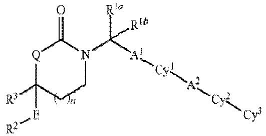 | |
| | | or in Zuang, bioorganic and medicinal chemistry, 2017, 25(14):3649-3657 | |
| | | 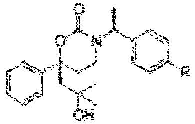 | |
| Pyrazoles | | Compounds of the following structure as described in PCT/EP/2007/052269 | |
| | | 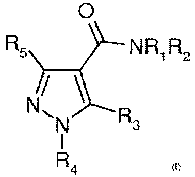 | |
| Alkyl-pyridazine | | Compounds of the following structures as described in PCT/EP/2007/056347 | |
| | | 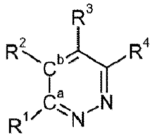 | |

According to a particular embodiment, a HSD11B1 inhibitor according to the invention is selected from adamantyltriazoles, arylsulfonamidothiazoles, anilinothiazolones, spirocyclic ureas as described in *Anagnostis et al., 2013, 62(1):21-33; Webster et al., 2017, supra.*

According to another particular embodiment, a HSD11B1 inhibitor according to the invention is selected from 3,4,5-trisubstituted-4-1,2,4-triazole, N-(thiazol-2-yl) benzenesulfonamide, S-(1-H-pyrazol-4-yl)thiophene-3-carboxamide, cyclohexylcarbamoyl spirocyclic ureas, arylsulfonamides, adamantanes, sulphonamides, carbamoyl, thiazolones and glycyrrhetinic acid derivatives as described above.
For example, according to a further particular embodiment, HSD11B1 inhibitors are selected from compounds and pharmaceutically acceptable salts thereof reported in Table 2 below:

**Table 2**

| **Common name** | **Structure** | **IUPAC name** |
|---|---|---|
| 10J (CAS number: 1009373-58-3) | 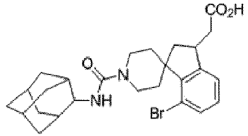 | (+/-)-2-(7-bromo-1'-((2-adamantyl) carbamoyl)-2,3-dihydrospiro [indene-1,4'-piperidine]-3-yl) acetic acid |
| PF-915275 (CAS number: 857290-04-1) | 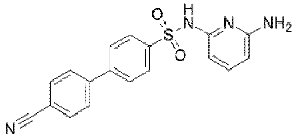 | *N*-(6-aminopyridin-2-yl)-4-(4-cyanophenyl) benzene sulfonamide |
| BVT2733 (CAS number: 376640-41-4) | 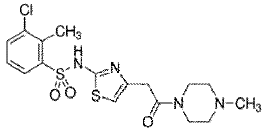 | 3-chloro-2-methyl-N-[4-[2-(4-methylpiperazin-1-yl)-2-oxoethyl]-1,3-thiazol-2-yl]benzenesulfonamide |
| BMS-823778 (CAS number: 1140898-87-8) | 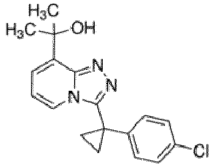 | 2-[3-[1-(4-chlorophenyl)cyclopropyl]-[1,2,4]triazolo[4,3-a]pyridin-8-yl]propan-2-ol |
| carbenoxolone (CAS number: 5697-56-3) | 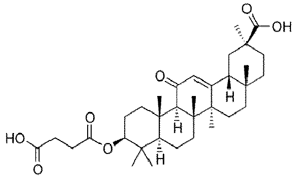 | (2S,4aS,6aR,6aS,6bR,8aR,10S,12aS,14bR )-10-(3-carboxypropanoyloxy)-2,4a,6a,6b,9,9,12a-heptamethyl-13-oxo-3,4,5,6,6a,7,8,8a,10,11,12,14b-dodecahydro-1H-picene-2-carboxylic acid |
| MK-0736 (CAS number 719272-79-4) | 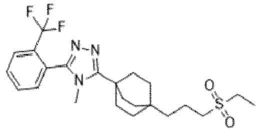 | 3-{4-[3-(ethanesulfonyl)propyl]bicyclo[2.2.2]octa n-1-yl}-4-methyl-5-[2-(trifluoromethyl)phenyl]-4H-1,2,4-triazole |
| AMG-221-BVT-83370 (CAS number 1095565-81-3) | 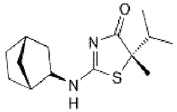 | (5*S*)-2-[[(1*S*,2*S*,4*R*)-2-bicyclo[2.2.1]heptanyl] imino]-5-methyl-5-propan-2-yl-1,3-thiazolidin-4-one |
| AZD8329 (CAS number 1048668-70-7) | 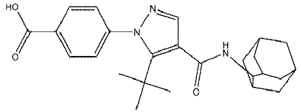 | 4-[4-(2-adamantylcarbamoyl)-5-*tert*-butyl pyrazol-1-yl]benzoic acid |
| ABT-384 (CAS number 868623-40-9) | 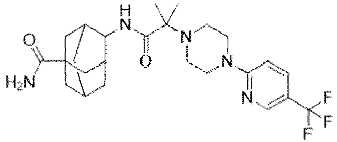 | 4-[[2-methyl-2-[4-[5-(trifluoromethyl)pyridin-2-yl]piperazin-1-yl]propanoyl]amino]adamantane-1-carboxamide |
| BVT-3498 (CAS number 376641-49-5) | 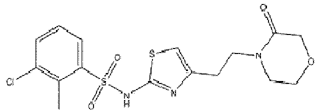 | 3-chloro-2-methyl-N-[4-[2-(3-oxomorpholin-4-yl)ethyl]-1,3-thiazol-2-yl]benzenesulfonamide |
| MK0916 (CAS number 633317-53-0) | 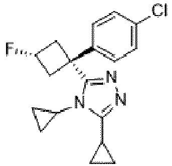 | 3-[1-(4-chlorophenyl)-3-fluorocyclobutyl]-4,5-dicyclopropyl-1,2,4-triazole |
| AZD4017 (CAS number 1024033-43-9) | 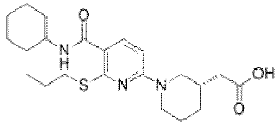 | 2-[(3*S*)-1-[5-(cyclohexylcarbamoyl)-6-propyl sulfanylpyridin-2-yl]piperidin-3-yl]acetic acid |
| HSD-016 (CAS number 946396-92-5) | 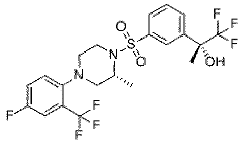 | (2R)-1,1,1-trifluoro-2-[3-[(2R)-4-[4-fluoro-2-(trifluoromethyl)phenyl]-2-methylpiperazin-1-yl] sulfonylphenyl]propan-2-ol |
| BI 135585 (CAS number 1114561-85-1) | 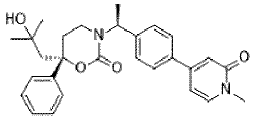 | (6R)-6-(2-hydroxy-2-methylpropyl)-3-[(1R)-1-[4-(1-methyl-2-oxopyridin-4-yl)phenyl]ethyl]-6-phenyl-1,3-oxazinan-2-one |
| INCB-13739 (CAS number 872506-67-7) | 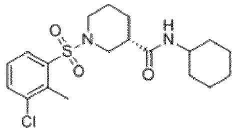 | (3S)-1-[(3-Chloro-2-methylphenyl)sulfonyl]-N-cyclohexyl-3-piperidinecarboxamide |
| UE2343 (CAS number 1346013-80-6) | 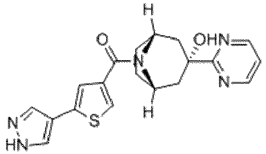 | [(1*R*,5*S*)-3-hydroxy-3-pyrimidin-2-yl-8-azabicyclo[3.2.1]octan-8-yl]-[5-(1*H-*pyrazol-4-yl)thiophen-3-yl]methanone |
| UE2316 | 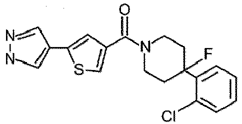 | [4-(2-chlorophenyl)-4-fluoro-1-piperidyl]-[5-(1H-pyrazol-4-yl)-3-thienyl]methanone |
| CRx-401 | 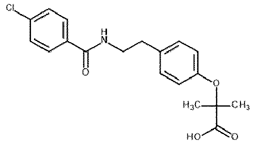 | 2-[4-[2-[(4-chlorobenzoyl)amino] ethyl]phenoxy]-2-methylpropanoic acid |

Further inhibitors can be identified by *in silico* models, as described in Liu et al., 2019, J. Chem. Inf. Model., 59, 3422-3436*.*
The expression "immunotherapy" includes strategies used to activate effector immune cells to increase the efficacy of the patient's own immune response against neoplastic cells (melanoma, lung carcinoma, glioblastoma, renal carcinoma, gastrointestinal stromal carcinoma and leukemia). Immunotherapy includes the use of cancer vaccines, oncolytic viruses, cell therapy and/or immune system modulators.
The expression "immune system modulators" covers small molecules and large molecules (peptides, chimeric proteins, antibodies, bispecific antibodies) that induce an anti-cancer immune response or that aim to neutralize immune suppressive mechanisms driven by T regulators cells, tumor associated macrophages, myeloid derived suppressor cells or immune checkpoints.
Vaccines can be used in an immunotherapy according to the invention and work against cancer by boosting the immune system's response to cancer cells. Therapeutic vaccines are different from vaccines that help prevent disease. Examples of therapeutic vaccines include sipuleucel-T (Topalian et al., 2011, J. Clin. Oncol., 29: 4828-36*, Dendreon*).
Oncolytic viruses represent a novel drug class in which native or modified viruses mediate tumor regression through selective replication within and lysis of tumor cells as well as induction of systemic antitumor immunity capable of eradicating tumor at distant, uninjected sites. An example of oncolytic viruses includes the herpesvirus-based treatment T-VEC (talimogene laherparepvec, Liu et al., Gene Therapy, 2003 10(4):292-303*, Amgen*).
Cell therapy includes treatments based on *in vitro* expanded and/or genetically modified lymphocytes (T cells or NK cells). For instance, T-cell transfer therapy, also called adoptive cell therapy, adoptive immunotherapy, or immune cell therapy includes tumor-infiltrating lymphocytes as starting material and genetically engineered CAR T-cells, which specifically recognize and kill tumor cells, such as described for the treatment of relapsed or refractory B-cell malignancies, such as B-cell non-Hodgkin lymphoma (NHL), acute lymphoblastic leukemia (ALL) and chronic lymphocytic leukemia (CLL) in pediatric and adult patients (Miliotou et al, 2018, Current pharmaceutical biotech., 19(1):5-18*.*)*.* Example of T cell transfer therapies includes tisagenlecleucel (Novartis, Forsberg et al., 2018, Ther Clin Risk Manag. 14:1573-1584 and Vairy, 2018, Drug Design, Development and Therapy, 12: 3885-3898*),* and axicabtagene ciloleucel (Gilead Sciences, Jain et al., 2018, Therapeutics and Clinical Risk Management, 14: 1007-1017)*.*
Immune system modulators enhance the body's immune response against cancer. Some of these agents affect specific parts of the immune system, whereas others affect the immune system in a more general way. This class includes cytokines (interferons, interleukins, granulocyte-macrophage colony-stimulating factor and granulocyte colony-stimulating factor), Bacillus Calmette-Guerin, immunomodulatory drugs (thalidomide, lenalidomide, pomalidomide, imiquimod) and chemotherapeutic agents that induce immunogenic cell death (cyclophosphamide, bleomycin, shikonin, anthracyclines).
Are included as immune system modulators monoclonal antibodies and antibody-derived constructs used as immunotherapy according to the invention, which are proteins designed to bind to specific targets on cancer cells. Some monoclonal antibodies and antibody-derived constructs mark cancer cells so that they will be better seen and destroyed by the immune system. Monoclonal antibodies may also be called therapeutic antibodies. For example, those monoclonal antibodies can be rituximab (Biogen, Genentech, Hoffmann-La Roche, Chugai Pharmaceuticals, Zenyaku Kogyo and AryoGen, DOI: 10.1016/j.trac.2013.02.014). For example, antibody-derived constructs can include short-chain variable antibody fragments (scFv) and can be bispecific T-cell engagers (BiTE) such as blinatumomab (Amgen, Wu et al., 2015, J. Hematol. Oncol., 8, 104)*.*
Are also included as immune system modulators the immune checkpoint blockers targeting PD-1 such as pembrolizumab (Merck, US 2010/0266617, DOI: 10.1158/1078-04), nivolumab (Bristol-Myers Squibb, WO 2006/121168), cemiplimab (Regeneron, DOI: 10.1158/1535-71), spartalizumab (PDR001, Novartis, US 9,683,048), camrelizumab (SHR1210, Jiangsu Hengrui Medicinem, https://doi.org/10.1007/s40265-019-01167-0), sintilimab (IBI308, Innovent Biologics and Eli Lilly, DOI: 10.1080/19420862.2019.1654303, tislelizumab (BGB-A317, BeiGene and Celgene Corp, US 8,735,553), toripalimab (JS 001, Shanghai Junshi Bioscience, Keam, 2019, Drugs 79, 573-578), AMP-224 (GlaxoSmithKline, Amplimmune, Infante et al. 2013, J Clin Oncol, 31, 3044), AMP-514, AstraZeneca, Naing et al. 2019, J. Immunotherapy Cancer 7, 225). Are also included the CTLA-4 inhibitors such as ipilimumab (Bristol-Myers Squibb, WO 2001/014424, EP1503794). Are also included the PD-L1 inhibitors atezolizumab (Tecentriq™, Roche, US 8,217,149, Powles et al., 2014, Nature, 515, 558-562), avelumab (Bavencio™, Pfizer, Merk KGaA, WO 2013/079174 A1), BMS-936559 (Bristol-Myers Squibb) and durvalumab (Imfinzi™, AstraZeneca, WO 2011/066389).
Are included as immune system modulators the immunoconjugates that are antibodies, peptides or proteins conjugated to a second molecule, which can include immune cell death inducer agents and toxins or immune system modulators (cytokines, Bacillus Calmette-Guérin and immunomodulatory drugs).
The term "pharmaceutically acceptable salts or complexes" refers to salts or complexes of the below-specified compounds of the invention. Examples of such salts include, but are not restricted, to base addition salts formed by reaction of compounds of the invention with organic or inorganic bases such as hydroxide, carbonate, bicarbonate or the like, of a metal cation such as those selected in the group consisting of alkali metals (sodium, potassium or lithium), alkaline earth metals (e.g. calcium or magnesium), or with an organic primary, secondary or tertiary alkyl amine. Other examples of such salts include, but are not restricted, to acid addition salts formed by reaction of compounds of the invention with organic or inorganic acids such as hydrochloric acid, hydrobromic acid, sulphuric acid, para-toluene sulfonic acid, 2-naphtalene sulfonic acid, camphosulfonic acid, benzene sulfonic acid, oxalic acid or the like.

### Combined use and combined compositions

The invention provides pharmaceutical or therapeutic agents as compositions and methods useful for the treatment of cancer.

According to a particular embodiment, the HSD11B1 inhibitors of the invention are to be used in combination with an immunotherapy selected from anti-cancer vaccine, adoptive cell therapy such as T-cell transfer therapy (inhibitors and genetic approaches), a small or a large molecules such as chemotherapies inducing immune cell death, anti-cancer monoclonal antibody, immunoconjugates, bispecific proteins, and cytokines.
According to a further particular aspect, the invention provides a use of a combination comprising at least one HSD11B1 inhibitor and at least one immune checkpoint inhibitor. According to a further particular aspect, an immune checkpoint inhibitor is selected from a PD-1 inhibitor, a CTLA-4 inhibitor and a PD-L1 inhibitor.
According to a further particular aspect, a PD-1 inhibitor is pembrolizumab or nivolumab. According to another aspect, the invention provides a use of a combination comprising at least one HSD11B1 inhibitor and at least one CTLA-4 inhibitor for the treatment of cancer, in particular of cancers associated with melanoma, lung carcinoma, glioblastoma, renal carcinoma, gastrointestinal stromal carcinoma and leukemia.
According to a further particular aspect, a CTLA-4 inhibitor is ipilimumab.
According to another aspect, the invention provides a use of a combination comprising at least one HSD11B1 inhibitor and at least one PD-L1 inhibitor for the treatment of cancer, in particular of cancers associated with melanoma, lung carcinoma, glioblastoma, renal carcinoma, gastrointestinal stromal carcinoma and leukemia.
According to a further particular aspect, a PD-L1 inhibitor is atezolizumab.
According to another particular aspect, the invention provides a use of a combination comprising at least one HSD11B1 inhibitor and at least one at least one anti-cancer vaccine.

According to another particular aspect, the invention provides a use of a combination comprising at least one HSD11B1 inhibitor and at least one anti-cancer monoclonal antibody.

According to another further aspect, is provided a pharmaceutical composition comprising a combination according to the invention and at least one more pharmaceutically acceptable carrier.

Compositions of this invention may further comprise one or more pharmaceutically acceptable additional ingredient(s) such as alum, stabilizers, antimicrobial agents, buffers, colouring agents, flavouring agents, adjuvants, and the like.
The compositions according to the invention, together with a conventionally employed adjuvant, carrier, diluent or excipient may be placed into the form of pharmaceutical compositions and may be employed as solids, such as tablets or filled capsules, or liquids such as solutions, suspensions, ointments, emulsions, elixirs, or capsules filled with the same, films or gels, all for oral use. The compositions may also be formulated as a dry product for reconstitution with water or other suitable vehicle before use.
Compositions of this invention as liquid formulations including, but not limited to, aqueous or oily suspensions, solutions, emulsions, syrups, and elixirs.
Such liquid preparations may contain additives including, but not limited to, suspending agents, emulsifying agents, non-aqueous vehicles and preservatives. Suspending agents include, but are not limited to, sorbitol syrup, methyl cellulose, glucose/sugar syrup, gelatin, hydroxyethyl cellulose, carboxymethyl cellulose, aluminum stearate gel, and hydrogenated edible fats. Emulsifying agents include, but are not limited to, lecithin, sorbitan monooleate, and acacia. Preservatives include, but are not limited to, methyl or propyl p-hydroxybenzoate and sorbic acid. Dispersing or wetting agents include but are not limited to poly(ethylene glycol), glycerol, bovine serum albumin, Tween®, Span®.
Further materials as well as formulation processing techniques and the like are set out in The Science and Practice of Pharmacy (Remington: The Science & Practice of Pharmacy), 22nd Edition, 2012, Lloyd, Ed. Allen, Pharmaceutical Press*, which is incorporated herein by* reference.
Solid compositions of this invention may be in the form of tablets or lozenges formulated in a conventional manner. For example, tablets and capsules for oral administration may contain conventional excipients including, but not limited to, binding agents, fillers, lubricants, disintegrants and wetting agents. Binding agents include, but are not limited to, syrup, acacia, gelatin, sorbitol, tragacanth, mucilage of starch and polyvinylpyrrolidone. Fillers include, but are not limited to, lactose, sugar, microcrystalline cellulose, maize starch, calcium phosphate, and sorbitol. Lubricants include, but are not limited to, magnesium stearate, stearic acid, talc, polyethylene glycol, and silica. Disintegrants include, but are not limited to, potato starch and sodium starch glycolate. Wetting agents include, but are not limited to, sodium lauryl sulfate. Tablets may be coated according to methods well known in the art.

In a particular embodiment, the invention provides a pharmaceutical formulation according to the invention for use as a medicament.

### Mode of administration

According to the invention, the HSD11B1 inhibitor or a pharmaceutical formulation thereof, are administered to an individual prior to, or simultaneously with an anti-cancer immunotherapeutic agent, for example concomitantly through the same formulation or separately through different formulations, in particular through different formulation routes. According to a particular aspect of the invention, a HSD11B1 inhibitor according to the invention and pharmaceutical formulations thereof is to be administered chronically (e.g. daily or weekly) for the duration of treatment and prior to the administration of an anti-cancer immunotherapeutic agent treatment.
According to another particular aspect of the invention, a HSD11B1 inhibitor according to the invention and pharmaceutical formulations thereof is to be administered concomitantly with an anti-cancer immunotherapeutic agent.
Compositions of this invention may be administered in any manner, including, but not limited to, orally, intravenously, intramuscularly, subcutaneously, parenterally, nasally, intralesionally or combinations thereof. Parenteral administration includes, but is not limited to subcutaneous and intramuscular. The compositions of this invention may also be administered in the form of an implant, which allows slow release of the compositions as well as a slow controlled i.v. infusion. In a particular embodiment, one or more HSD11B1 inhibitor is administered orally. The compounds of a combination of this invention may be administered independently by any manner by oral route such as sublingually, via buccal administration, including to the mucosal surfaces of the oral cavity including the gingiva, the floor of the oral cavity, cheeks, lips, tongue, teeth.

According to a particular embodiment, compositions of this invention may be administered by intravenous or intra-muscular or sub-cutaneous injection.

The dosage administered, as single or multiple doses, to an individual will vary depending upon a variety of factors, including pharmacokinetic properties, patient conditions and characteristics (age, body weight, health, body size), extent of symptoms, frequency of treatment and the effect desired.

### Patients

In an embodiment, patients according to the invention are patients suffering from a cancer.

In a particular embodiment, patients according to the invention are patients suffering from a cancer selected from melanoma, lung carcinoma, glioblastoma, renal carcinoma, gastrointestinal stromal carcinoma and leukemia.
In a particular embodiment, patients according to the invention are patients suffering from tumors of the skin, melanoma, lung, pancreas, breast, colon, rectum, brain, laryngeal, ovarian, prostate, colorectal, head, neck, testicular, lymphoid, marrow, bone, sarcoma, renal, gastrointestinal tract, sweat gland, and the like tissues.
In a particular embodiment, patients according to the invention are patients suffering from melanoma.
In another particular embodiment, patients according to the invention are patients suffering from lung carcinoma.
In a particular embodiment, patients according to the invention are patients suffering from glioblastoma.
In a particular embodiment, patients according to the invention are patients suffering from renal carcinoma.
In a particular embodiment, patients according to the invention are patients suffering from gastrointestinal stromal carcinoma.
In a particular embodiment, patients according to the invention are patients suffering from leukemia.
In another further embodiment, patients according to the invention are patients suffering from breast and renal cancer.

### Use and methods according to the invention

In a particular embodiment, the use of compounds of the invention or methods of treatment using those increases responsiveness to immunotherapy.
In another particular embodiment, the combined use of compounds of the invention and methods using this combination induce the level of IFN gamma.
Therefore, the use of inhibitory agents or methods that would silence or down-regulate or inhibit HSD11B1 would be beneficial to subject's responsiveness to immunotherapeutic treatment of cancer.
According to another aspect of the invention, is provided a method for treating a subject who is suffering from a cancer, said method comprising administering a therapeutically effective amount of an agent inducing HSD11B1 inhibition or any suitable pharmaceutically acceptable formulation thereof, in a subject in need thereof in combination with immunotherapy.
According to another further embodiment, such agent inducing HSD11B1 inhibition can be agents that knock out or knock down HSD11B1 at the genetic level using methods such as CRISPR-Cas9 *technology* (Pickar-Olivier and Gerbach, 2019, Nature Reviews Molecular Cell Biology, 20, 490-50*)* and small interference RNA (Chakraborty et al., Mol Ther Nucleic Acids. 2017, 8: 132-143*.)* or gene therapy (Dunbar et al., 2018, Science 359, 6372)*.*
In another embodiment, the invention provides a method of identifying agents useful in the potentiation of immunotherapy, said method comprising the following steps:
- exposing at least one immune cell such as dendritic cells, macrophages, neutrophils, T cells with cancer cells (alone or in co-culture) with HSD11B1 substrates (e.g. cortisone or 11-DHC);
- contacting said at least one cell with at least one candidate agents;
- identifying agents that are able to inhibit HSD11B1 in said cells.

Examples illustrating the invention will be described hereinafter in a more detailed manner and by reference to the embodiments represented in the Figures.

### EXAMPLES

The following abbreviations refer respectively to the definitions below:
**BMDC** (Bone marrow derived dendritic cells); **DMEM** (Modified Eagle's minimal essential medium); **DMSO** (Dimethyl Sulfoxyde); **FBS** (Fetal bovine serum); **GFP** (green fluorescent protein); **GMCSF** (Granulocyte-macrophage colony-stimulating factor); **RPMI** (Roswell Park Memorial Institute).

### Example 1: Assessing the effect of inhibition of HSD11B1 in combination with immunotherapy for the use as treatment of cancer in an antigen recall assay

Response to immunotherapy rely on critical biological steps including antigen presentation and T cell activation. Therefore, the therapeutic potential of combining immunotherapy with HSD11B1 inhibition was investigated in an antigen recall assay as described below.
Antigen recall assay is the gold standard *in vitro* method to assess the bioactivity of immune checkpoint inhibitors because it measures at the same time the potential of antigen presenting cells to activate T cell and the actual T cell activation, taking into account thereby the two critical mechanisms required for an optimal response to immunotherapy. The level of IFNγ secreted in this human immune cell-based assay is considered as a good indicator of the T cells activation to immunomodulatory molecules. Cryopreserved human peripheral blood mononuclear cell (PBMC) isolated from cytomegalovirus (CMV) positive heathy donors were thawed in a 37°C water bath and seeded in round bottom 96 well plates at 2 X 10⁵ cells/well in 150 µL final volume of RPMI media supplemented with 10% fetal bovin serum, 1% non-essential amino acids, 0.01M Hepes, 1% Penicilin/Streptomycin, 1% L-glutamine and 50 ng/ml cortisone). PBMC were stimulated with 1 µg/ml CMV antigen (Astarte) and with 1 µg/ml anti PD-1 (G4K) or isotype control (Biovision) and with 1 µM of a HSD11B1 inhibitors (10J, carbenoxolone and BMS-823778 from Sigma and PF-915275 from R&D Systems) or DMSO control. Plates were incubated at 37°C, 6% CO₂. After 6 days, supernatants were assayed for IFNγ by ELISA (Biolegend).
As illustrated in **Fig. 1****,** the level of IFNγ measured in PBMC supernatant was increased in the presence of the anti-PD-1 compared to isotype control. Importantly, co-treatment with the anti-PD-1 and the HSD11B1 inhibitors significantly increased the level on IFNγ when compared to anti-PD-1 or HSD11B1 inhibitor alone. This experiment therefore provide evidence that inhibition of HSD11B1 is effective to improve the efficacy of immunotherapy.

### Example 2: Assessing the effect of inhibition of HSD11B1 in combination with immunotherapy for the use as treatment of cancer in a cell growth assay

Another critical biological mechanism required for an optimal response to immunotherapy of cancer is the capacity of T cells to kill the tumor target cells. The therapeutic potential of combining immunotherapy with HSD11B1 inhibition for the treatment of cancer was therefore assessed in a murine T cell-mediated tumor cell-killing assay.
Wild type C57BL/6J murine bone marrow progenitors cells were maintained with 20 ng/ml GMCSF (Peprotech) to induce differentiation into bone marrow derived dendritic cells (BMDCs) for 6 days. At day 6, 2 X 10⁴ BMDCs/well together with 2 X 10³ Renca tumor cells/well were seeded in flat bottom 96 well plate in RPMI 1640 supplemented with 10% FBS, 1% Penicillin/streptomycin, 1% L-glutamine, 50 mM betamercaptoethanol and 0.5% of sodium pyruvate, 32 ng/ml R848 (Invivogen) and 25 µg/ml ovalbumin (Invivogen). After 24 hours, cell supernatant was removed and 1x10⁵ OT-I CD8 T cells/well were added in very low endotoxin RPMI 1640 medium supplemented with 10% fetal bovine serum, 1% L-glutamine, 1% penicillin-streptomycin, 1% sodium-pyruvate, 1% glutamine, 1%, non-essential amino acids and 50 mM beta-mercaptoethanol. After 24 hours, 5x10³ H2kb-recombined; SEQ ID NO: 1 SIINFEKL (Invivogen) loaded-GFP-expressing Renca cells/well were added. The growth of GFP Renca cells was measured and quantified in real time by cell imaging using the Incucyte technology. 200 ng/ml 11-DHC (US Biological), 10 µM HSD11B1 inhibitor (BMS-823778 from Sigma) or vehicle DMSO and anti-PD-1 (Invivogen) or isotype control (Invivogen) were maintained from day 6 until the end of the experiment.
As illustrated in **Fig. 2****,** the combination of an HSD11B1 inhibitor with an anti-PD-1 (mouse) inhibited the growth of tumor cells more efficiently than the single treatments.
These data provide therefore strong evidences that the inhibition of HSD11B1 is effective to improve the efficacy of immunotherapy for the treatment of cancer.

### Example 3: Genetic deletion of HSD11B1

In order to assess the utility of genetic loss of function particularly for adoptive cell therapy (CAR T cell), genetic deletion of HSD11B1 can be performed using CRISPR-Cas9 *technology* (Pickar-Olivier and Gerbach, 2019, Nature Reviews Molecular Cell Biology, 20, 490-50) in mouse T cells. Isolated CD8 T cell from OT-1 mouse are grown for 24 hours with 5 ng/ml IL-7 (R&D system) in RPMI 1640 medium supplemented with 10% fetal bovine serum, 1% L-glutamine, 1% sodium-pyruvate, 1% glutamine, 1%, non-essential amino acids and 50 mM beta-mercaptoethanol. After 24h, cells are electroporated with Cas9/crRNA complex targeting HSD11B1 (crRNA sequence 1: 5'-GCTGGGCACAAGTTCGAGTTAGG-3' (SEQ ID NO: 2); crRNA sequence 2: 5'-ATGGTGGTCATCTTGGTCGTAGG-3' (SEQ ID NO: 3)) with Neon electroporation (Thermofisher) as described by Seki et al. 2018, JEM, 5;215(3):985-997*.* T cells are then activated with ovalbumin loaded BMDCs as described in Example 2. Three days post activation, 5x10³ H2kb-recombined; SIINFEKL (SEQ ID NO: 1) loaded-GFP-expressing Renca cells/well are added. The growth of GFP Renca cells is then measured and quantified in real time by cell imaging using the Incucyte technology. 200 ng/ml 11-DHC (US Biological), 10 µM HSD11B1 inhibitor (BMS-823778 from Sigma) or vehicle DMSO and anti-PD-1 (Invivogen) or isotype control (Invivogen) are maintained from day 2 post T cell activation until the end of the experiment.

### SEQUENCE LISTING

**SEQ ID NO: 1 -** Ovalbumin peptide (chicken)
   SIINFEKL
**SEQ ID NO: 2** - crRNA sequence (gRNA targeting mouse hsd11b1) 5' -GCTGGGCACAAGTTCGAGTTAGG-3'
**SEQ ID NO: 3 -** crRNA sequence (gRNA targeting mouse hsd11b1) 5' -ATGGTGGTCATCTTGGTCGTAGG-3'

## Claims

1. A HSD11B1 inhibitor for use in the treatment of a cancer, wherein said HSD11B1 inhibitor is to be administered in combination with immunotherapy.

2. A HSD11B1 inhibitor for use according to claim 1 in combination with at least one immunotherapeutic agent selected from a cancer vaccine, oncolytic virus and an immune system modulator.

3. A HSD11B1 inhibitor for use according to claim 1 in combination with cell therapy.

4. A HSD11B1 inhibitor for use according to claim 1 or 2 in combination with at least one immune checkpoint inhibitor.

5. A HSD11B1 inhibitor for use according to claim 4, wherein said at least one immune checkpoint inhibitor is selected from a PD-1inhibitor, an CTLA-4 inhibitor and an PD-L1 inhibitor.

6. A HSD11B1 inhibitor for use according to any one of claims 1 to 5 in combination with at least one PD-1inhibitor.

7. A HSD11B1 inhibitor for use according to claim 6, wherein said at least one PD-1 inhibitor is pembrolizumab.

8. A HSD11B1 inhibitor for use according to any one of claims 1 to 5 in combination with at least one CTLA-4 inhibitor.

9. A HSD11B1 inhibitor for use according to claim 1 or 2 in combination with at least one anti-cancer vaccine.

10. A HSD11B1 inhibitor for use according to any one of claims 1 to 9, wherein said HSD11B1 inhibitor is selected from an adamantyltriazoles, an arylsulfonamidothiazole, an anilinothiazolone, spirocyclic urea and carbenoxolone.

11. A HSD11B1 inhibitor for use according to any one of claims 1 to 10, wherein said HSD11B1 inhibitor is selected from: 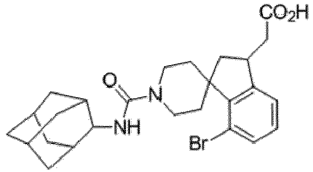 (10J or (+/-)-2-(7-bromo-1'-((2-adamantyl)carbamoyl)-2,3-dihydrospiro[indene-1,4'-piperidine]-3-yl)acetic acid); 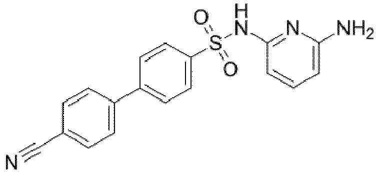 (PF-915275 or N-(6-aminopyridin-2-yl)-4'-cyanobiphenyl-4-sulfonamide); 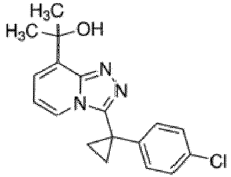 (BMS-823778 or 2-[3-[1-(4-chlorophenyl)cyclopropyl]-[1,2,4]triazolo[4,3-a]pyridin-8-yl]propan-2-ol); and 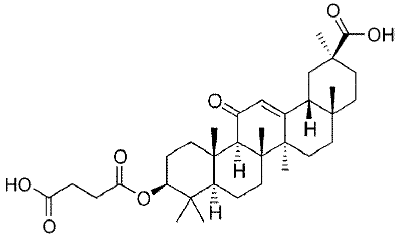 (carbenoxolone or (2S,4aS,6aR,6aS,6bR,8aR,10S,12aS,14bR)-10-(3-carboxypropanoyloxy)-2,4a,6a,6b,9,9,12a-heptamethyl-13-oxo-3,4,5,6,6a,7,8,8a,10,11,12,14b-dodecahydro-1H-picene-2-carboxylic acid) or any pharmaceuticaly acceptable salts thereof.

12. A HSD11B1 inhibitor for use according to any one of claims 1 to 11, wherein cancer is selected from melanoma, lung carcinoma, glioblastoma, renal carcinoma, gastrointestinal stromal carcinoma and leukemia.

13. A pharmaceutical composition comprising at least one HSD11B1 inhibitor, at least one agent useful in immunotherapy and at least one agent useful in immunotherapy and at least one pharmaceutically acceptable carrier.

14. A pharmaceutical composition according to claim 13, wherein said HSD11B1 inhibitor is selected from 10J, PF-915275, BMS-823778 and carbenoxolone.

15. A pharmaceutical composition according to any one of claims 13 to 14 for use as a medicament

16. A method of identifying agents useful in the potentiation of immunotherapy, said method comprising the following steps:
- exposing at least one immune cell such as dendritic cell, macrophage, neutrophil, T cell with cancer cell with at least one HSD11B1 substrate;
- contacting said at least one cell with at least one candidate agent;
- identifying agents that are able to inhibit HSD11B1 in said cells.
